# EUROPEAN PATENT APPLICATION

(11) **EP 2 806 367 A1**
(43) Date of publication of application: **26.11.2014**
(21) Application number: 14168888.7
(22) Date of filing: 19.05.2014
(51) Int. Cl.: G06F 19/00

(54) **Information structuring system**

(30) Priority: 20.05.2013 JP 2013105743
(71) Applicant: Hitachi Ltd., Tokyo 100-8280 (JP)
(72) Inventor: Kido, Kunihiko, Chiyoda-ku, Tokyo 100-8280 (JP); Yui, Shuntaro, Chiyoda-ku, Tokyo 100-8280 (JP)
(74) Representative: Moore, Graeme Patrick

(57) **Abstract**

The present invention periodically and exhaustively extracts analysis dimension candidates from text data, such as medical literatures disclosed on the Internet. A name of disease, a medical agent, a checkup, and an operation included in actual clinical data are linked to the analysis dimension candidates. In the analysis dimension candidates, clinically important candidates and non-important candidates including extraction errors are mixed. To distinguish the candidates, weighting is provided to the link. First, a weight is made large when a level of an evidence of a medical literature from which an analysis dimension candidate is extracted is high. In literature groups of each name of disease, the degree of cooccurrence between a word of an analysis dimension candidate, and a word related to a medical agent/checkup/operation is calculated, and the weight of the link is made larger according to the magnitude of the degree of cooccurrence.

## Description

### CROSS-REFERENCE TO RELATED APPLICATIONS

This application claims the priority of Japanese Patent Application No. 2013-105743, filed on May 20, 2013, which is incorporated herein by reference in its entirety.

### BACKGROUND OF THE INVENTION

### 1. Field of the Invention

The present invention relates to structuring of clinical data and a method of analyzing clinical data, for a database related to clinical information.

### 2. Description of the Related Art

As a background technology in the field of this technology, there is JP-2005-108248-A. In this document, there is description related to a medical assist system that includes a knowledge database accumulating clinical data and basic research data, decomposes both pieces of information as knowledge elements, and links and reorganizes relevance among the elements by weighting.

### SUMMARY OF THE INVENTION

When clinical data is analyzed from the clinical aspect, dimensions of the analysis are diverse, and it is difficult to determine them in advance. Examples of the dimensions of an analysis include a complication, the size of a cancer, the number of cancers, a dose of a medical agent, and the number of administration. Under the present circumstances, with respect to a specific disease, it is typical to limit the dimensions based on a clinical study plan and to construct a cube, instead of general and exhaustive data warehouse construction for clinical study. Meanwhile, diversity of the analysis dimensions means conditions for similar case search are diverse. That is, it is necessary that, for each individual case, a searcher examines a condition to characterize the case based on clinical knowledge, and includes the condition in a search sentence. Therefore, it is difficult to stylize the search sentence without narrowing down an object to be searched or a range. In a case of a relational database, it is necessary to perform search based on an SQL that is a search language after becoming knowledgeable about a table structure of the database. However, end users such as doctors who are not experts of the database cannot be often expected to make full use of the SQL sentence.

The clinical data is configured from a name of disease, a prescription, an operation, a checkup, and clinical data, such as a checkup result. These clinical data can be arranged and integrated according to attribute information, such as an object patient, a date of execution, and a data of recording. However, information of association based on a medical sense, such as a relation between a prescription, a checkup, and a technique, and a name of disease adaptable therefor may be often lacked. When an analysis data set is created in clinical study, it is typical that an analyzer manually collects related data in consideration of relevance between clinical data based on medical knowledge. Names of disease, medical agents, techniques, and checkup items are huge, and confirmation work of them takes a lot of time.

An information structuring system that performs information structuring using a database that stores medical knowledge information including medical concept information, the degree of cooccurrence of the medical concept information, and literature rating information of medical literature information of an acquisition source of the medical concept information, the information structuring system includes:
a clinical information input reception unit configured to receive an input of a plurality of pieces of clinical information; and
a link generation unit configured to generate link information that associates the plurality of pieces of clinical information each other by providing weight information including the degree of cooccurrence and the literature rating information, using medical knowledge information.

According to the present invention, clinical data are associated based on medical knowledge related to relevance between clinical data. In addition, in the association between the clinical data, a weight is given from the aspect of the degree of attention of researchers based on an evidence level and the degree of cooccurrence of the medical literature, which is an acquisition source of the medical knowledge. Therefore, data can be narrowed down based on the degree of importance according to an analysis purpose of the searcher. For example, when the searcher has an interest in an analysis by an analysis dimension widely acknowledged in an academic society, or the like, the data can be narrowed down according to relevance having a high weight based on an evidence level of a medical literature. Further, when the searcher wishes to collect data having high significance of study but a low evidence level, the data can be narrowed down based on relevance having a high degree of cooccurrence.

These data are exhaustively collected, and clinical data is structured based on the analysis dimension. In addition, regarding the relevance between an analysis dimension and actual clinical data, the weight is given from the degree of attention of researchers based on an evidence level of a medical literature and the degree of cooccurrence. Therefore, data necessary for an analysis purpose of the searcher can be easily searched based on exhaustively prepared analysis dimensions. For example, when the searcher has an interest in an analysis by a analysis dimension widely acknowledged in an academic society, or the like, the data can be narrowed down to an analysis dimension having a high weight based on an evidence level of a medical literature, and collected. Further, when the searcher wishes to collect data having a low evidence level but high significance of study, the data may just be narrowed down to an analysis dimension having a high degree of cooccurrence, and collected.

### BRIEF DESCRIPTION OF THE DRAWINGS

Fig. 1 is a system configuration diagram related to a first embodiment of the present invention;
Fig. 2 is a program configuration diagram related to the first embodiment of the present invention;
Fig. 3 is a program configuration diagram related to the first embodiment of the present invention;
Fig. 4 is a processing flow of a program related to the first embodiment of the present invention;
Fig. 5 is a processing flow of a program related to the first embodiment of the present invention;
Fig. 6 is an example of a table in which a patient ID and clinical information are associated;
Fig. 7 is an example of a table in which clinical information is managed;
Fig. 8 is an example of a table in which relation between pieces of clinical information is managed;
Fig. 9 is an example of a dictionary table for extracting a medical concept from a medical literature;
Fig. 10 is an example of a table in which a medical knowledge management table is managed;
Fig. 11 is an example of a table in which a rating of a medical literature is managed;
Fig. 12 is a table describing types of clinical study;
Fig. 13 is an example of a medical literature;
Fig. 14 is a screen example related to medical knowledge generation processing;
Fig. 15 is a screen example related to link generation processing;
Fig. 16 is a screen example related to search processing;
Fig. 17 is an example of a table in which a pre-aggregation value is managed;
Fig. 18 is a program configuration diagram related to the first embodiment of the present invention;
Fig. 19 is a processing flow of a program related to the first embodiment of the present invention; and
Fig. 20 is a processing flow of a program related to the first embodiment of the present invention.

### DESCRIPTION OF THE PREFERRED EMBODIMENTS

Hereinafter, embodiments of the present invention will be described with reference to the drawings.

### [First Embodiment]

Medical literature information of the present invention is electronic data including text data, such as medical papers, diagnostic treatment guidelines, and medical textbooks, in which knowledge related to diagnostic treatment is written. The medical paper includes a title, a date of publication, a body, an abstract, and keywords related to the content of the body. Further, the medical concept includes medical terms, such as a name of disease, a symptom, a name of medicine, a name of checkup, and a unit, and an equality/inequality configured from a combination of the medical terms.

The present invention periodically and exhaustively extracts analysis dimension candidates from text data, such as medical literatures disclosed on the Internet. Then, the present invention links data, such as a name of disease, a medical agent, a checkup, and an operation included in actual clinical data with the analysis dimension candidates. The analysis dimension candidates include information related to a side effect, inequalities related to the magnitude/the number/numerical values, and a temporal relation, for each name of disease, or a medical agent/checkup.

Here, in the analysis dimension candidates, candidates that are clinically important and candidates that are not important including an extraction error are mixed. To distinguish these candidates, weighting is performed with respect to the link in the following aspect. First, a weight is made large when a level of an evidence of a medical literature from which an analysis dimension candidate is extracted is high. This is because the analysis dimension candidate of a medical literature having a high evidence level can be estimated to have a high degree of recognition in the academic society. For example, a medical literature related to a meta-analysis of a randomization comparative test has a highest evidence level, and the degree of importance of an analysis dimension candidate included in the literature is high. Next, a paper that includes any one of randomization comparative tests has a second highest evidence level.

In literature groups of name of diseases, the degree of cooccurrence between a word of an analysis dimension candidate and a word related to a medical agent/checkup/operation is calculated, and a weight of the link is made larger according to the magnitude of the degree of cooccurrence. This is because an analysis dimension candidate examined in many papers can be estimated to be of highly interest to researchers.

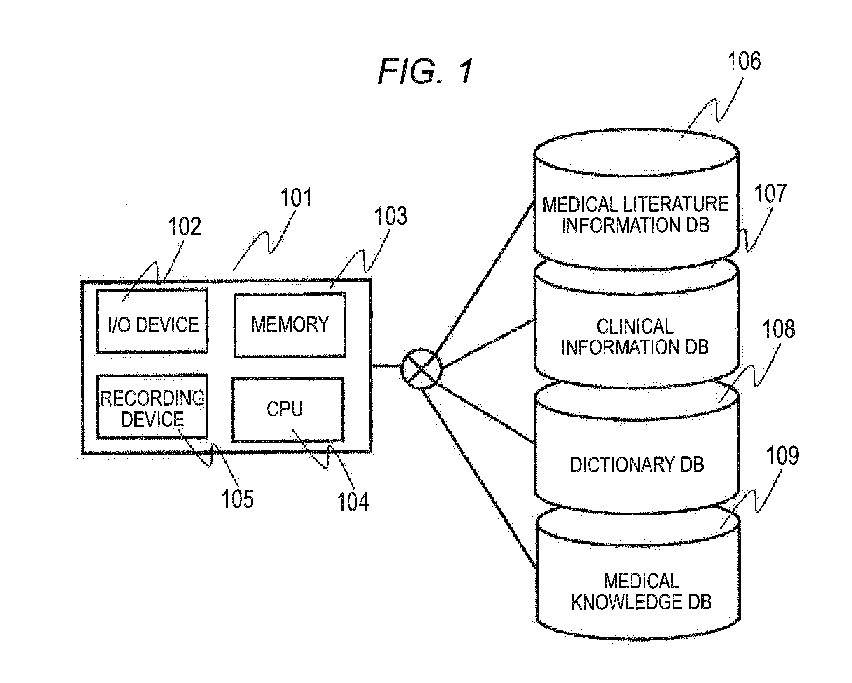

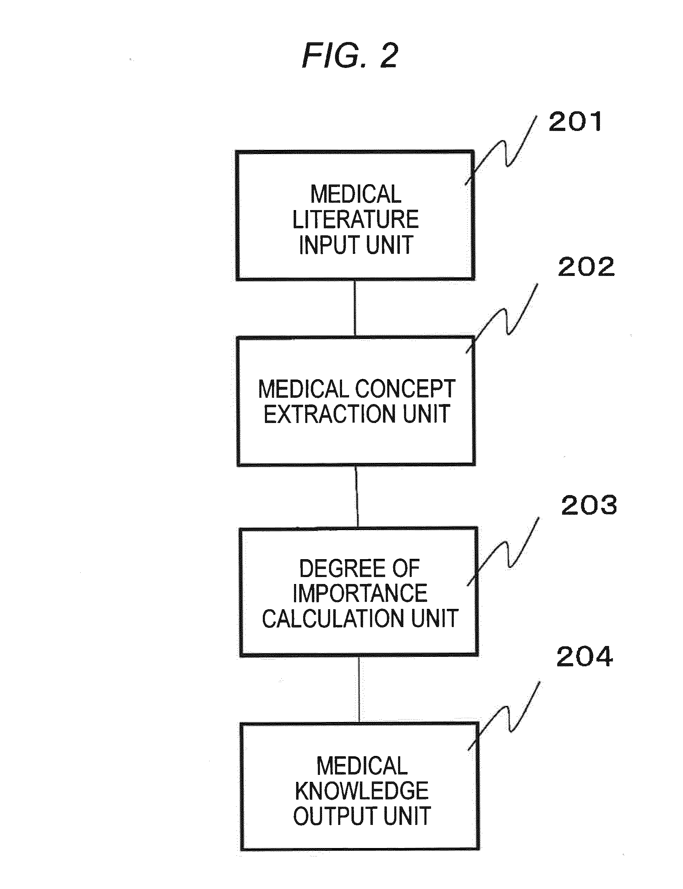

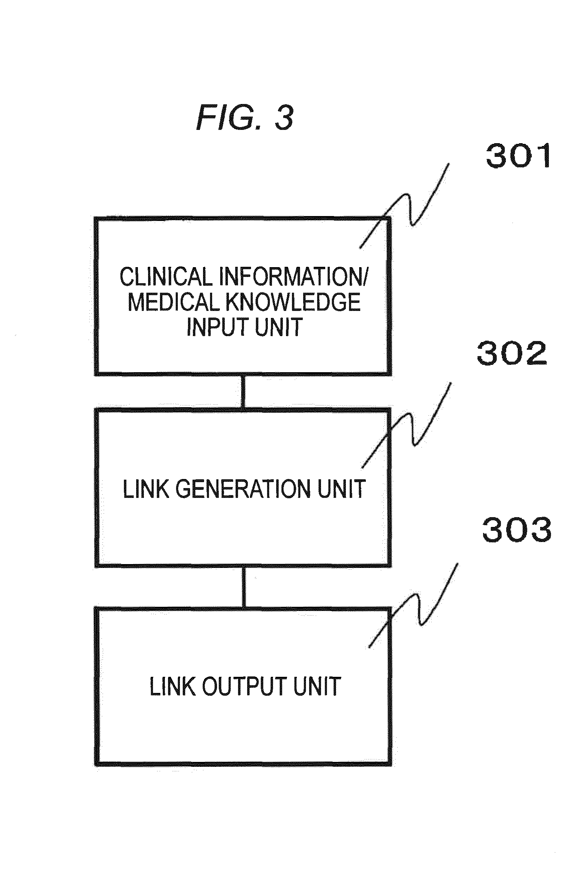

Fig. 1 illustrates a hardware configuration of the present embodiment. A computer 101 includes a memory 103, a storage device 105, a CPU 104, and an I/O device 102. Further, as external storage devices, a medical literature information DB 106, a clinical information DB 107, a dictionary DB 108, and a medical knowledge DB 109 are connected to the computer 101, and perform an input/output of data with the computer 101 through the I/O device 102. Figs. 2 and 3 illustrate program configuration diagrams of the present embodiment. Fig. 2 is a configuration of a program that generates information of the medical knowledge DB 109 based on data of the medical literature information DB 106. The program is configured from a clinical literature input unit 201, a medical concept extraction unit 202, a degree of importance calculation unit 203, and a medical knowledge output unit 204. The program is loaded from the storage device 105 to the memory 103 through the I/O device 102, and executed by the CPU 104. Fig. 3 illustrates a configuration of a program that generates a link of weighting between data regarding clinical information of the clinical information DB 107, based on medical knowledge of the medical knowledge DB 109. The program is configured from a clinical information/medical knowledge input unit 301, a link generation unit 302, and a link output unit 303. The program is loaded from the storage device 105 to the memory 103 through the I/O device 102, and executed by the CPU 104.

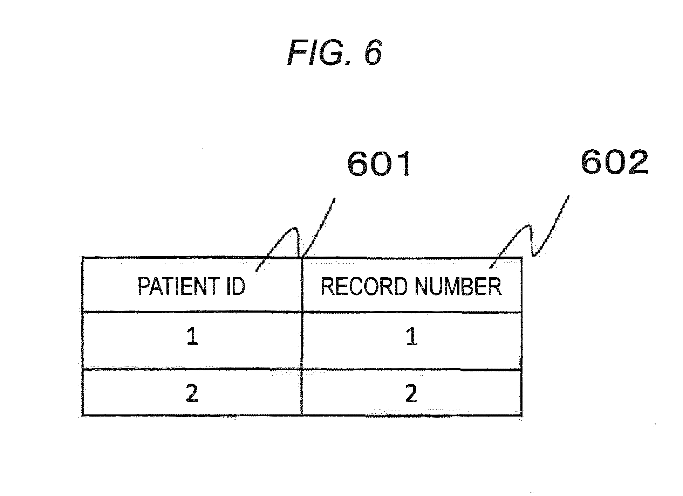

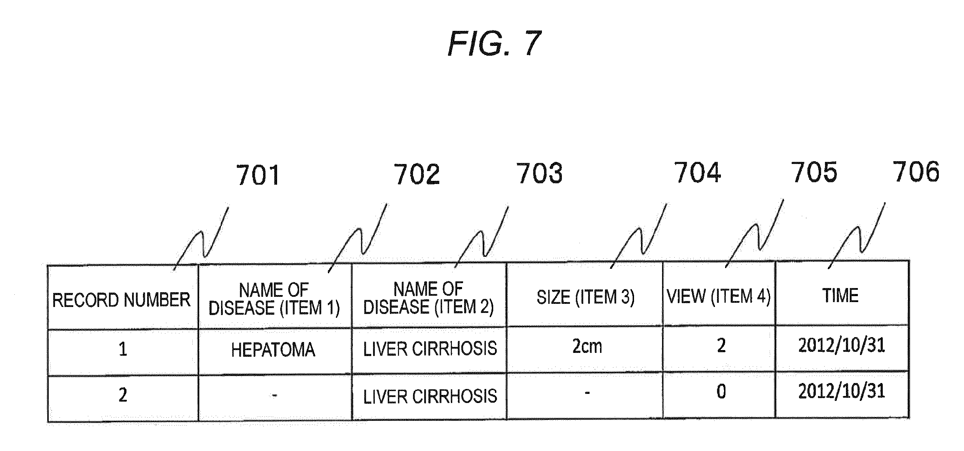

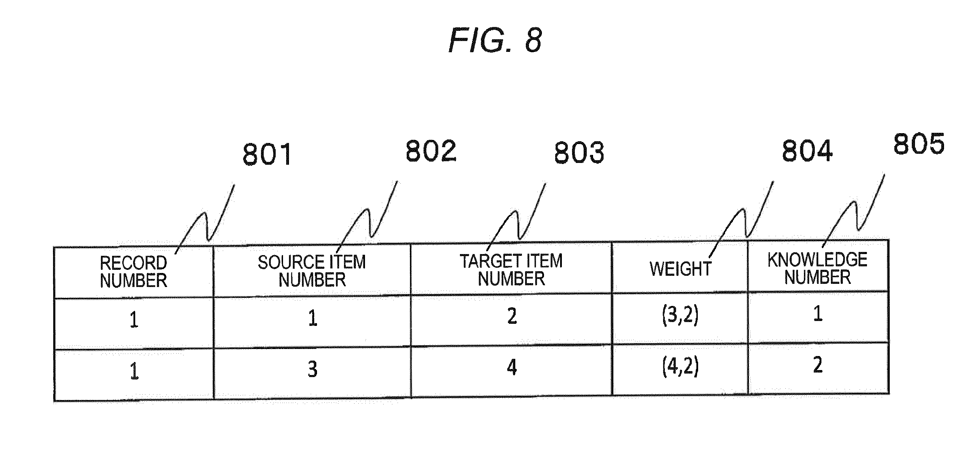

Figs. 6, 7, 8, 9, 10, and 11 illustrate configurations of tables used in the present embodiment. Fig. 6 is a correspondence table between a patient ID and clinical information, and the table is made of a patient ID 601 and a record number 602. Fig. 7 is a clinical information table, and the table is made of a record number 701, a name of disease (item 2) 702, a name of disease (item 2) 703, a size (item 3) 704, and a dimension (item 4) 705. Here, the reason why there are two names of disease is that a present system can deal with complications. A plurality of the name of disease items may be further included according to the number of complications. When the record number 602 of Fig. 6 refers to the record number 701 of Fig. 7, the patient ID and the clinical information are associated. Fig. 8 is a link table, in which a relation between the items of the clinical information is recorded. The table is made of a record number 801, a source item number 802, a target item number 803, a weight 804, and a knowledge number 805.

A source item and a target item mean a start point and an end point, such as a cause → an effect, a general concept → a more specific concept, or a name of current disease → a name of related disease, including a cause and an effect in a cause and effect relation, a general concept and a more specific concept in a conceptual inclusion relation, or a name of current disease and a name of related disease in advance of symptom.

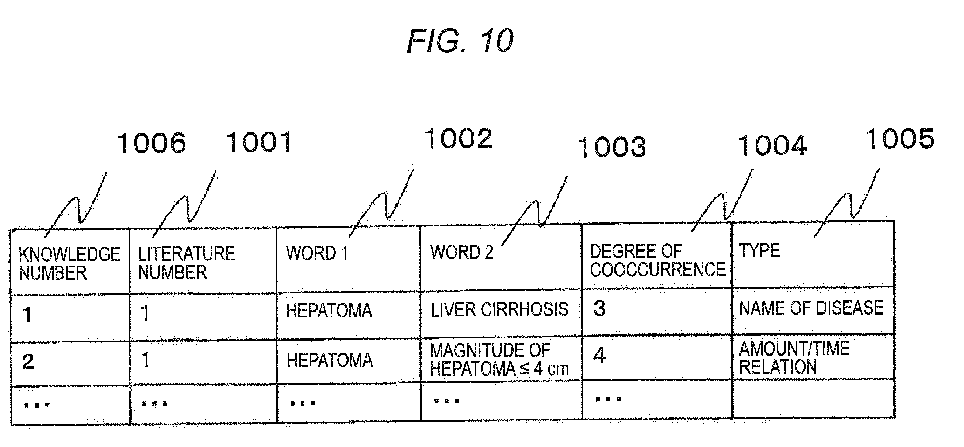

First, the record number 801 identifies a record of the clinical information table by referring to the record number of Fig. 7. If there is a link between the item 3 and the item 4, "3" is recorded in the source item number 802, "4" is recorded in the target item number 803, and a value vector (a, b) of a weight of the link is recorded in the weight 804. As described below, the first component a of the value vector (a, b) is rating information of a literature, and the second component b is defined as the degree of cooccurrence. Further, the knowledge number 805 is an identification number related to medical knowledge of the value vector of the weight of the link, and records a knowledge number of a corresponding record of the medical knowledge management table of Fig. 10.

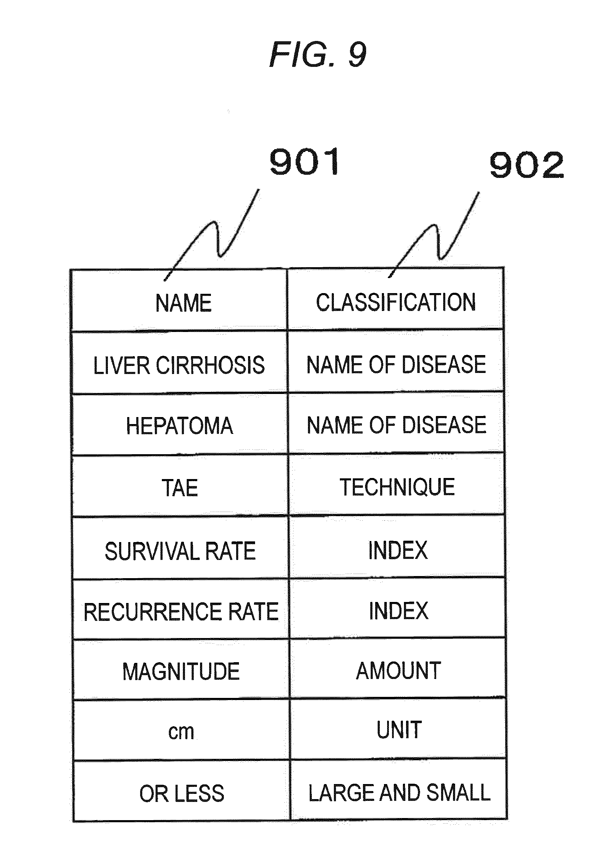

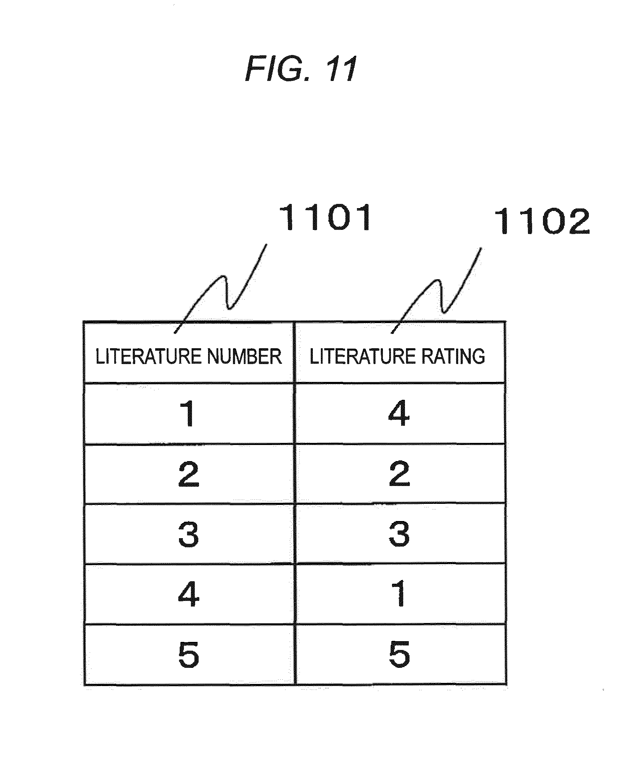

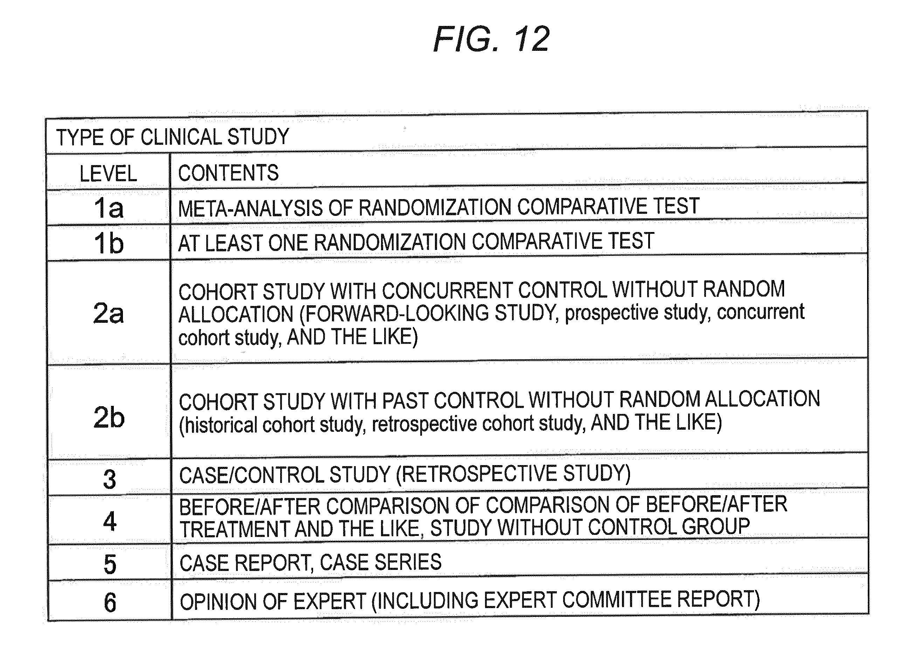

Fig. 9 is a dictionary table. The table is used for extracting a medical concept from a medical literature, and is made of a name 901 and classification 902. In the name 901, words of the medical concept to be extracted from a medical literature are recorded. In the classification 902, classification of the words is recorded. This classification includes a name of disease, a name of technique, a name of index, and a name of medicine. Fig. 10 is a medical knowledge management table. The table is used to manage a medical concept extracted from a medical literature and its degree of cooccurrence, and is made of a literature number 1001, a word (1) 1002 and a word (2) 1003 that are a pair of cooccurrence words, and the degree of cooccurrence 1004 of the cooccurrence words. Fig. 11 is a table in which rating of each literature is recorded, and the table is made of a literature number 1101, and a literature rating 1102. The dictionary table can be updated, and may be updated through a network, or the like. In the present embodiment, as the literature rating, levels of clinical study of Fig. 12 are employed. These levels can be changed, and may be changed through a network, or the like.

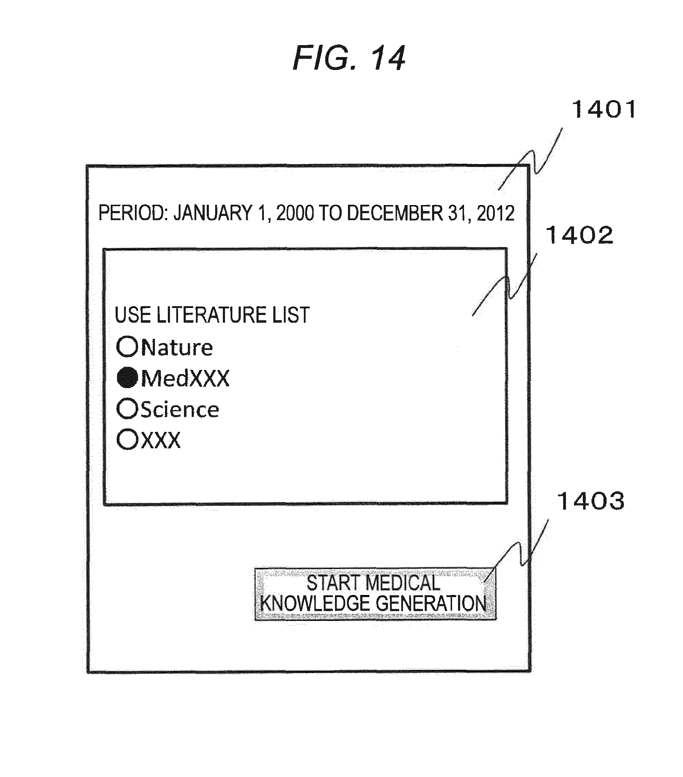

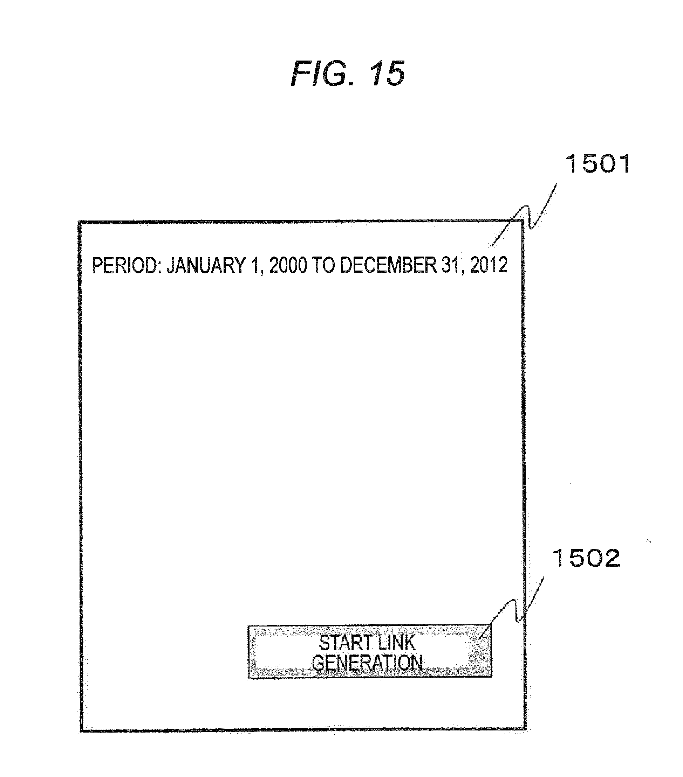

Figs. 14 and 15 are screen examples used in the present embodiment. Fig. 14 illustrates a screen used in the program that generates the medical knowledge illustrated in Fig. 2. An area 1401 is an area where the program specifies a period to be processed with respect to a medical literature stored in the medical literature information DB 106. An area 1402 is an area in which the program specifies a literature DB to be processed with respect to a medical literature stored in the medical literature information DB 106. A button 1403 is used to start processing of the program. As described above, a literature in a specific period is selected and information structuring is performed, so that a wide variety of demands for the structuring can be responded.

Fig. 15 illustrates a screen used in the program that generates the link illustrated in Fig. 3, and the screen is made of a period 1501 through which a period of clinical information that is an object of link generation is specified, and a link generation start button 1502.

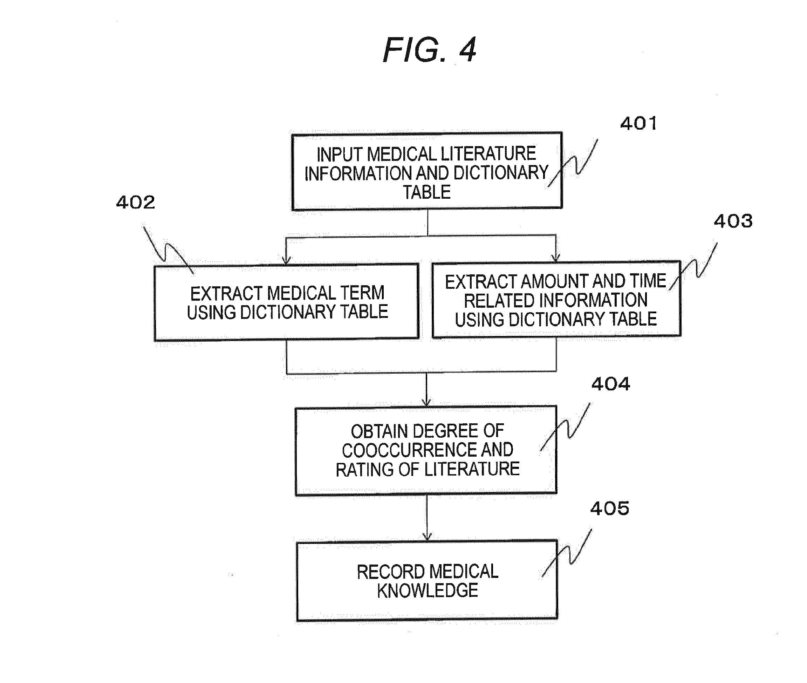

Fig. 4 illustrates a processing flow of the program of Fig. 2. When the medical knowledge generation start button 1403 on the screen of Fig. 14 is clicked, the program is started.

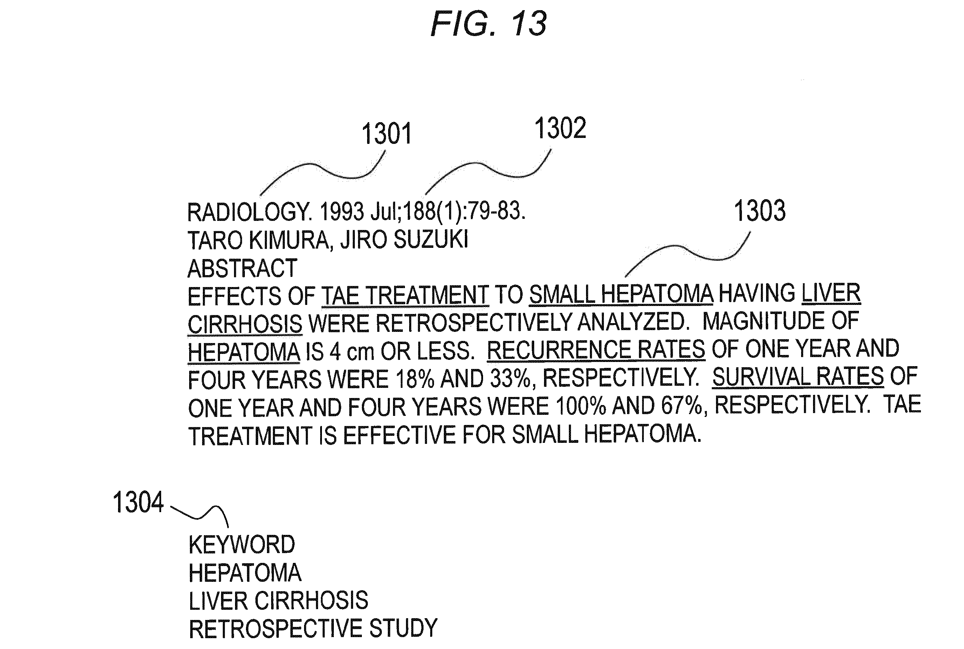

In step S401, a literature DB of medical literature and medical literatures of a period specified through the screen of Fig. 14 are taken in from the medical literature information DB 106 to the memory 103 through the I/O device 102. Fig. 13 is an example of a medical literature, and the medical literature is made of a literature title 1301, a date of publication 1302, an abstract 1303, and keywords 1304. Similarly, the dictionary table illustrated in Fig. 9 is taken in from the dictionary DB 108 to the memory 103 through the I/O device 102.

In step S402, medical terms that are examples of the medical concept are extracted from the abstract of the medical literature based on the name 901 related to the classification 902 of a name of disease, a technique, and an index, with respect to each record in the dictionary table. The underlined portions in the abstract 1303 of Fig. 13 are examples of the medical terms extracted based on the dictionary table of Fig. 9. Similarly, in step S403, amount and time relation information that is an example of the medical concept is extracted based on the name 901 related to the classification 902 of the name of disease, an amount, large and small, a unit, with respect to each record of the dictionary table. Here, the amount and time relation is extracted. However, other classification items of the medical concept may be used. Describing using the example of Fig. 13, "the magnitude of hepatoma is 4 cm or less" is extracted. The hepatoma is the "name of disease", the magnitude is the "amount", cm is the "unit", and or less is "large and small", and these words are sandwiched by a preposition of "of, a verb of "is", and a figure. Therefore, this sentence is determined to be the amount and time relation information. In step S404, a rating of the literature is identified from the keywords of the literature information. Next, the degree of cooccurrence is obtained about the medical terms and the amount and time relation information extracted in step S403. Here, the degree of cooccurrence of an item A and an item B is defined to be the number of literatures that concurrently include the item A and the item B. In step S405, regarding the rating of the literature obtained in step S404, the rating is registered in the literature rating 1102 and the literature number 1101 of the literature table of Fig. 11. As for the degree of cooccurrence, the literature number, a pair of cooccurrence words, and its degree of cooccurrence are registered in the medical knowledge management table of Fig. 10. The medical knowledge information that is weighted by the rating using the medical literature information is generated in this way, and highly accurate information structuring can be assisted. Further, information related to a medical care can be structured from various analysis dimensions using the classification items. Further, the medical knowledge information weighted

by the degree of cooccurrence or the literature rating is generated using the medical literature information, and the highly accurate information structuring can be assisted.

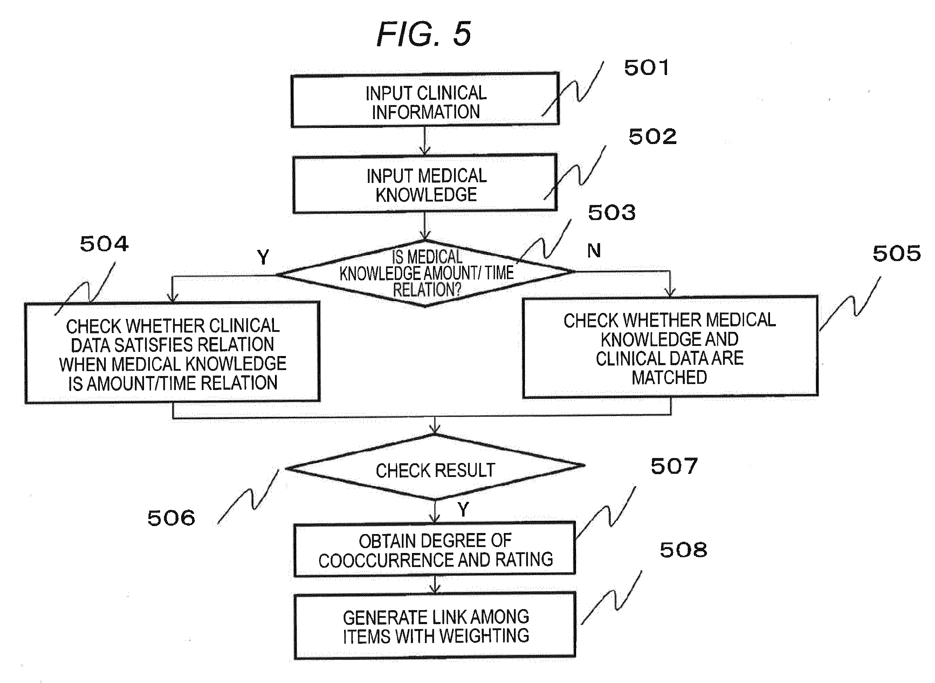

Fig. 5 illustrates a processing flow of the program of Fig. 3. When the link generation start button 1502 on the screen of Fig. 15 is clicked, the program is started.

In step S501, clinical data in the period 1501 specified through the screen of Fig. 15 is taken from the clinical information DB 107 to the memory 103 through the I/O device 102. To be specific, the time 706 in the clinical information table of Fig. 7 is referred, and all of records that match the period 1501 specified through the screen of Fig. 15 are searched, and are taken in to the memory 103 through the I/O device 102. The clinical information in a specific period is selected in this way, and the information structuring is performed, so that a wide variety of demands for the structuring can be responded.

In step S502, medical knowledge is taken from the medical knowledge DB 109 to the memory 103 through the I/O device 102. To be specific, all of records are taken from the medical knowledge management table of Fig. 10 to the memory 103 through the I/O device 102. Further, the period 1501 may be determined after an input to set the period is received.

In step S503, the records are acquired one at a time from all of the records of the medical knowledge taken in in step S502, and whether the medical knowledge is the amount/time relation is checked from the type 1005 of the medical knowledge management table of Fig. 10. To be specific, the medical knowledge that includes an equality sign, or an inequality sign is determined to be the amount/time relation.

If the medical knowledge is not the amount/time relation, in step S505, whether the word 1 of the reference sign 1002 and the word 2 of the reference sign 1003 match any of the name of disease (item 1), the name of disease (item 2), and the size (item 3) (the reference signs 702 to 704) of the records of the clinical data acquired in step S501. In step S506, matching of YES/NO is checked, and if YES, the processing proceeds to step S507.

Meanwhile, if the medical knowledge is the amount/time relation, in step S504, whether the relation of the clinical data satisfies the equality or the inequality of the medical knowledge is checked. For example, regarding the medical knowledge of the "hepatoma" and the "magnitude of hepatoma ≤ 4 cm", if the name of disease (item 1) 702 is the "hepatoma" and the size (item 3) is 2 cm, the clinical data matches the inequality relation of the medical knowledge. Here, the clinical data matches the inequality relation, a medical knowledge number of the medical knowledge is obtained from the medical knowledge number 1006 of the medical knowledge management table of Fig. 10, and the medical knowledge number is re-entered to the dimension of the reference sign 705 of Fig. 7. As described above, the information related to a medical care can be structured from various analysis dimensions using the classification items.

In step S506, a check result of steps S504 and S505 are examined, and if YES, the processing proceeds to step S507.

In step S507, a rating of a medical literature of a literature number that includes the medical knowledge is obtained from Fig. 11, and the degree of cooccurrence of the word 1 of the reference sign 1002 and the word 2 of the reference sign 1003 is obtained from Fig. 10. For example, in the case of the "hepatoma" and the "magnitude of hepatoma ≤ 4 cm" that are the medical knowledge related to a medical literature of the literature number 1, it can be found that the rating of the literature is 4 from Fig. 11, and the degree of cooccurrence is 3 from Fig. 10.

In step S508, a record is generated in the link table of Fig. 8, and the value vector (a, b) that is a weight of a link is generated from the degree of cooccurrence and the rating obtained in step S505, together with the record number 801, the source item number 802, and the target item number 803, and are registered in the weight 804 of the record,. Further, the knowledge number of the medical knowledge that is an object to be processed in step S507 is registered in the knowledge number 805. Here, the source item number 802 is determined from the name of disease (item 1) (reference sign 702), the name of disease (item 2) (reference sign 703), or the size (item 3) (reference sign 704) of the record of the clinical data that matches the word 1 of the reference sign 1002. To be specific, the source item number 802 is 1 when the name of disease (item 1) (reference sign 702) is matched, the source item number 802 is 2 when the name of disease (item 2) (reference sign 703) is matched, or the source item number 802 is 3 when the size (item 3) (reference sign 704) is matched. Note that, as for the matching of the size (item 3) (reference sign 704), when the reference sign 704 satisfies the amount/time relation of the medical knowledge number of the dimension (item 4) (reference sign 705), the matching is determined.

Similarly, the target item number 803 is determined from the name of disease (item 1) (reference sign 702), the name of disease (item 2) (reference sign 703), or the dimension (item 4) (reference sign 705) of the record of the clinical data that matches the word 2 of the reference sign 1003. To be specific, the target item number 803 is 1 when the name of disease (item 1) (reference sign 702) is matched, the target item number 803 is 2 when the name of disease (item 2) (reference sign 703) is matched, or the target item number 803 is 4 when the dimension (item 4) (reference sign 705) is matched. Note that, as for the matching of the dimension (item 4) (reference sign 705), when the size (item 3) (reference sign 704) satisfies the amount/time relation of the medical knowledge number of the dimension (item 4) (reference sign 705), the matching is determined. As described above, the highly accurate information structuring can be performed by putting weight information using the degree of cooccurrence and the literature rating.

Next, search processing will be described.

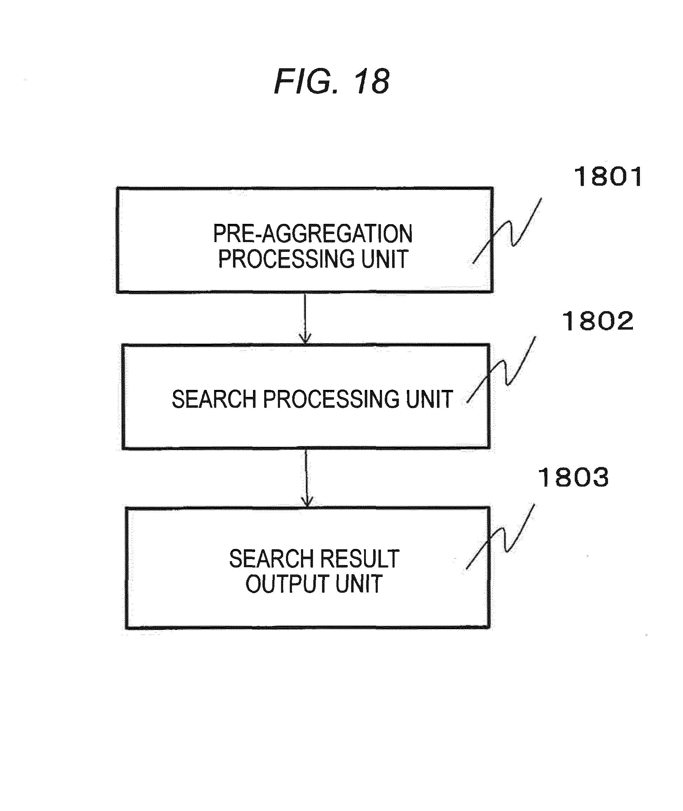

Fig. 18 illustrates a program configuration diagram of search processing in the present embodiment. This program is made of a pre-aggregation processing unit 1801, a search processing unit 1802, and a search result output unit 1803. The program is loaded from the storage device 105 to the memory 103 through the I/O device 102, and executed by the CPU 104.

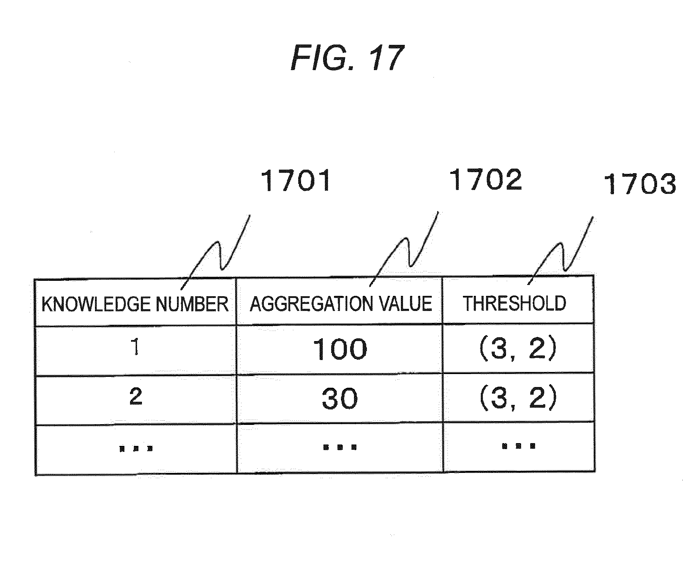

Fig. 17 illustrates a pre-aggregation table, in which numerical values obtained by aggregating the clinical information related to the dimensions of an analysis based on the medical knowledge in advance are managed. The table is made of a knowledge number 1701, an aggregation value 1702, and a threshold 1703. The knowledge number 1701 is an identification number of the medical knowledge that is the basis of a dimension of an analysis. The aggregation value 1702 is an aggregation value of the clinical information that matches the medical knowledge.

For example, a case where the aggregation value of the knowledge number 1 in Fig. 17 is 100 will be described. The knowledge number 1 is knowledge that hepatoma and liver cirrhosis are related from Fig. 10, and the aggregation value 100 of the knowledge number 1 indicates there are 100 cases in which hepatoma and liver cirrhosis coexist in actual clinical data.

Further, in the threshold 1703, a threshold related to a weight of the medical knowledge that becomes an object to be aggregated is managed.

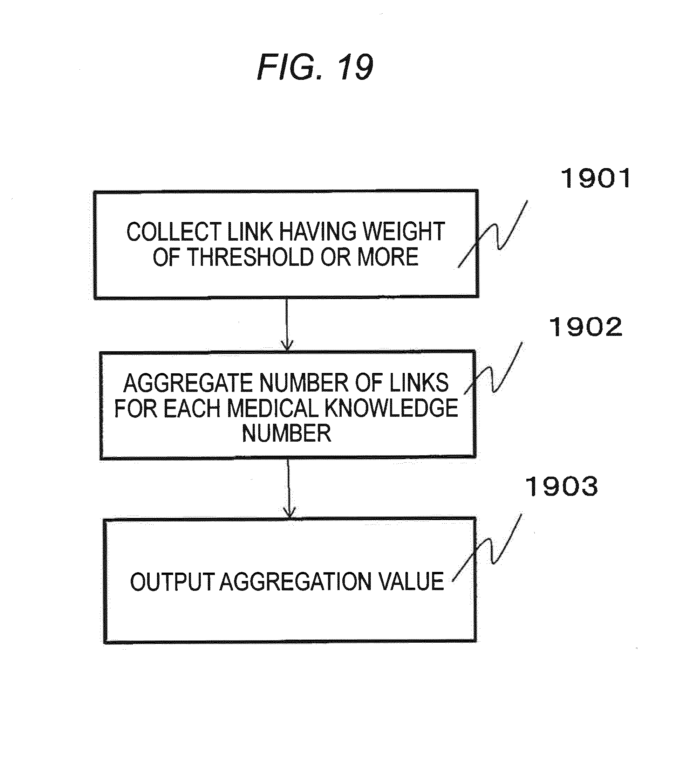

Here, a processing flow of the pre-aggregation processing unit 1801, which is processing of creating the table of Fig. 17, will be described with reference to Fig. 19. This processing is periodically executed, such as once a day. The processing may be nonperiodically executed based on some trigger. The knowledge number of the pre-aggregation table and the record of the medical knowledge management table of the knowledge number provide a dimension of an analysis exhaustively extracted from the medical literature. By aggregating the number of pieces of the clinical information corresponding to the dimension of an analysis in advance, aggregation calculation can be omitted in actual search, and the analysis can be performed at a high speed. In step S1901, the link table of Fig. 8 is searched for records in which the weight 804 has a predetermined threshold or more. In step S1902, the number of records is aggregated for each different knowledge number 805 regarding the records searched in step S1901. In step S1903, the aggregation value for each knowledge number aggregated in step S1902 is recorded in the knowledge number 1701, the aggregation value 1702, and the threshold 1703 of Fig. 17, together with the knowledge number and the threshold used in step S1801. As described above, an efficient analysis can be performed by aggregating and segmenting structured information.

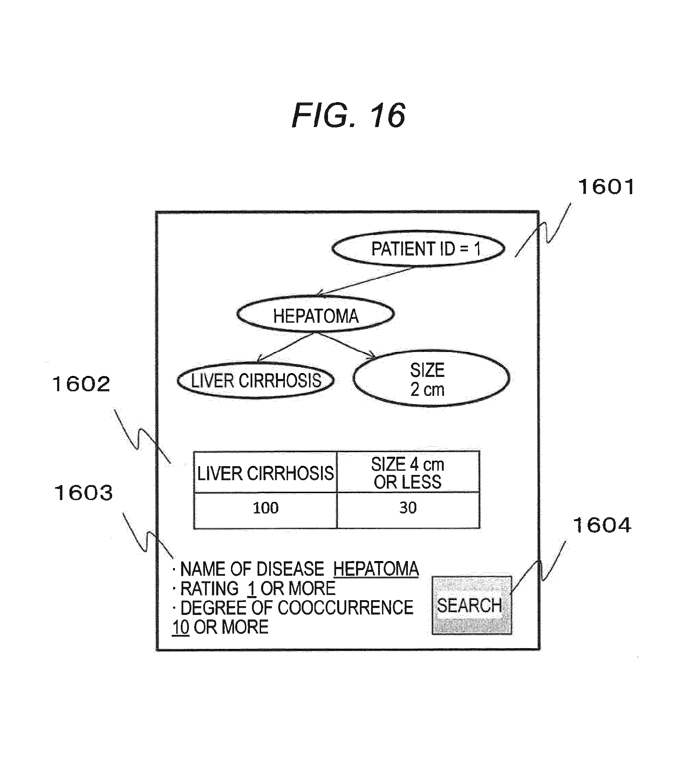

Fig. 16 is a screen example used in the present embodiment. The screen is made of an area 1601 that displays a graph of a search result, a display area 1602 of a pre-aggregation value based on a dimension of an analysis according to the medical knowledge, an input area 1603 of a search condition, and a search button 1604.

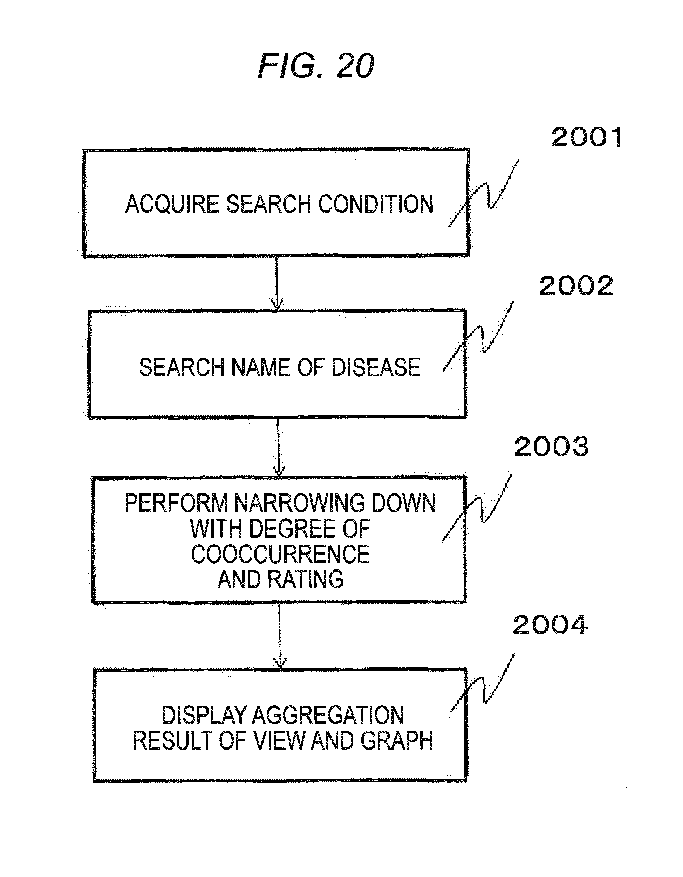

Next, a processing flow of the search processing unit 1802 and the search result output unit 1803 will be described with reference to Fig. 20.

With a click of the search button 1604 of Fig. 16, the program is started. In step S2001, the name of disease, the rating, and the degree of cooccurrence specified in the input area 1603 related to the search condition of Fig. 16 are acquired.

In step S2002, regarding the name of disease acquired in step S2001, all of records that match the name of disease of the reference sign 702 or the reference sign 703 of the clinical information table of Fig. 7 are searched. In step S2003, records of the link table of Fig. 8 that match the record number 701 of each record acquired in step S2002 are obtained with reference to the record number 801. Next, the value vectors of the weight 804 of the records are taken out, and the records are further narrowed down to records having a larger rating and degree of cooccurrence than those acquired in step S2001.

In step S2004, with respect to the records narrowed down in step S2003, a record that matches the record number 602 in the correspondence table of the patient ID and the clinical information of Fig. 6 is searched based on the record number, and the patient ID 601 is obtained. Here, a graph structure for display is created, and is displayed in the area 1601 of Fig. 16. This graph structure provides a link to the source item number 802 of the link table using the patient ID as a route. Next, the source item number 802 and the target item number 803 are linked.

In actual display, a medical concept corresponding to the source item number 802 and the target item number 803 is obtained from registration content of a corresponding item number in the records of the clinical information table of Fig. 7 with which the record number is matched.

For example, in the link table of Fig. 8, a record having the record number of 1, the source item number of 1, and the target item number of 2 is focused. In the record having the record number of 1 of Fig. 7, the item 1 is the name of disease and the "hepatoma" is registered, and the item 2 is the name of disease and the "liver cirrhosis" is registered. From the above, a link of "hepatoma → liver cirrhosis" is provided. Note that, in a case of the item number of 4, that is, the dimension of the reference sign 704, a record is searched based on the knowledge number 1006 of the medical knowledge management table of Fig. 10, based on the knowledge number recorded in the dimension (item 4) (reference sign 704), and description content of the word 2 of the reference sign 1003 is acquired and displayed,. For example, in the link table of Fig. 8, a record having the record number of 1, the source item number of 3, and the target item number of 4 is focused. In the record having the record number of 1 of Fig. 7, the item 3 is the size and is 2 cm, and the item 4 is the dimension and the knowledge number 2 is registered. In the medical knowledge management table of Fig. 10, the word 2 of the record of the knowledge number 2 is the "magnitude of hepatoma ≤ 4 cm". From the above, a link of "size 2 cm → magnitude of hepatoma ≤ 4 cm" is provided.

As described above, as a search condition, only the name of disease, the rating, and the degree of cooccurrence have been specified as examples. However, in a search result, related data is displayed in the form of a graph structure based on the dimension of an analysis introduced from the medical knowledge related to the name of disease. Therefore, the searcher can easily display information necessary for an analysis without specifying a condition related to a dimension of an analysis. Further, the information to be displayed can be narrowed down by the rating and the degree of cooccurrence. For example, when the searcher has an interest in an analysis by an analysis dimension widely acknowledged in an academic society, or the like, the searcher narrows down information to an analysis dimension having a high rating of medical literature. Further, when the searcher wishes to collect at a having high significance of study, the searcher can narrow down the information to an analysis dimension having a high degree of cooccurrence.

Next, regarding the knowledge number 805 of the records of the link table used for creation of the graph structure, records that match the knowledge number 1701 of the pre-aggregation table of Fig. 17 are searched.

If there is a record that matches the knowledge number 1701, the record is displayed in the area 1602 of Fig. 16 in a display format based on the name of the knowledge number and the aggregation value 1702. As described above, the number of pieces of clinical information corresponding to the dimension of an analysis is displayed at a high speed, without especially specification by the searcher. Note that the name of the knowledge number is obtained by searching the medical knowledge management table of Fig. 10 for a record that matches the knowledge number, and acquiring the word 1003. The link information aggregated using the degree of cooccurrence and the literature information is searched in this way, and an efficient analysis can be performed.

A clinical information database structured for clinical study is provided to medical institutions, such as hospitals. Accordingly, the clinical study, such as study of effective treatment method and the like, is facilitated, and a contribution to the development of the medical technology is made.
[Fig. 1]
   102 I/O DEVICE
   103 MEMORY
   105 RECORDING DEVICE
   106 MEDICAL LITERATURE INFORMATION DB
   107 CLINICAL INFORMATION DB
   108 DICTIONARY DB
   109 MEDICAL KNOWLEDGE DB
[Fig. 2]
   201 MEDICAL LITERATURE INPUT UNIT
   202 MEDICAL CONCEPT EXTRACTION UNIT
   203 DEGREE OF IMPORTANCE CALCULATION UNIT
   204 MEDICAL KNOWLEDGE OUTPUT UNIT
[Fig. 3]
   301 CLINICAL INFORMATION/MEDICAL KNOWLEDGE INPUT UNIT
   302 LINK GENERATION UNIT
   303 LINK OUTPUT UNIT
[Fig. 4]
   401 INPUT MEDICAL LITERATURE INFORMATION AND DICTIONARY TABLE
   402 EXTRACT MEDICAL TERM USING DICTIONARY TABLE
   403 EXTRACT AMOUNT AND TIME RELATED INFORMATION USING DICTIONARY TABLE
   404 OBTAIN DEGREE OF COOCCURRENCE AND RATING OF LITERATURE
   405 RECORD MEDICAL KNOWLEDGE
[Fig. 5]
   501 INPUT CLINICAL INFORMATION
   502 INPUT MEDICAL KNOWLEDGE
   503 IS MEDICAL KNOWLEDGE AMOUNT/TIME RELATION?
   504 CHECK WHETHER CLINICAL DATA SATISFIES RELATION WHEN MEDICAL KNOWLEDGE IS AMOUNT/TIME RELATION
   505 CHECK WHETHER MEDICAL KNOWLEDGE AND CLINICAL DATA ARE MATCHED
   506 CHECK RESULT
   507 OBTAIN DEGREE OF COOCCURRENCE AND RATING
   508 GENERATE LINK AMONG ITEMS WITH WEIGHTING
[Fig. 6]
   601 PATIENT ID
   602 RECORD NUMBER
[Fig. 7]
   701 RECORD NUMBER
   702 NAME OF DISEASE (ITEM 1) HEPATOMA
   703 NAME OF DISEASE (ITEM 2) LIVER CIRRHOSIS
   704 SIZE (ITEM 3)
   705 VIEW (ITEM 4)
   706 TIME
[Fig. 8]
   801 RECORD NUMBER
   802 SOURCE ITEM NUMBER
   803 TARGET ITEM NUMBER
   804 WEIGHT
   805 KNOWLEDGE NUMBER
[Fig. 9]
   901 NAME
      LIVER CIRRHOSIS
      HEPATOMA
      SURVIVAL RATE
      RECURRENCE RATE
      MAGNITUDE
      OR LESS
   902 CLASSIFICATION
      NAME OF DISEASE
      TECHNIQUE
      INDEX
      AMOUNT
      UNIT
      LARGE AND SMALL
[Fig. 10]
   1006 KNOWLEDGE NUMBER
   1001 LITERATURE NUMBER
   1002 WORD 1
      HEPATOMA
   1003 WORD 2
      LIVER CIRRHOSIS
      MAGNITUDE OF HEPATOMA ≤ 4 cm
   1004 DEGREE OF COOCCURRENCE
   1005 TYPE
      NAME OF DISEASE
      AMOUNT/TIME RELATION
[Fig. 11]
   1101 LITERATURE NUMBER
   1102 LITERATURE RATING
[Fig. 12]
   TYPE OF CLINICAL STUDY
   LEVEL
   CONTENTS
   1a META-ANALYSIS OF RANDOMIZATION COMPARATIVE TEST
   1b AT LEAST ONE RANDOMIZATION COMPARATIVE TEST
   2a COHORT STUDY WITH CONCURRENT CONTROL WITHOUT RANDOM ALLOCATION (FORWARD-LOOKING STUDY, prospective study, concurrent cohort study, AND THE LIKE)
   2b COHORT STUDY WITH PAST CONTROL WITHOUT RANDOM ALLOCATION (historical cohort study, retrospective cohort study, AND THE LIKE)
   3 CASE/CONTROL STUDY (RETROSPECTIVE STUDY)
   4 BEFORE/AFTER COMPARISON OF COMPARISON OF BEFORE/AFTER TREATMENT AND THE LIKE, STUDY WITHOUT CONTROL GROUP
   5 CASE REPORT, CASE SERIES
   6 OPINION OF EXPERT (INCLUDING EXPERT COMMITTEE REPORT)
[Fig. 13]
   1301 RADIOLOGY
   TARO KIMURA, JIRO SUZUKI
   ABSTRACT
   1303 EFFECTS OF TAE TREATMENT TO SMALL HEPATOMA HAVING LIVER CIRRHOSIS WERE RETROSPECTIVELY ANALYZED. MAGNITUDE OF HEPATOMA IS 4 cm OR LESS. RECURRENCE RATES OF ONE YEAR AND FOUR YEARS WERE 18% AND 33%, RESPECTIVELY. SURVIVAL RATES OF ONE YEAR AND FOUR YEARS WERE 100% AND 67%, RESPECTIVELY. TAE TREATMENT IS EFFECTIVE FOR SMALL HEPATOMA.
   1304 KEYWORD
   HEPATOMA
   LIVER CIRRHOSIS
   RETROSPECTIVE STUDY
[Fig. 14]
   1401 PERIOD: JANUARY 1, 2000 TO DECEMBER 31, 2012
   1402 USE LITERATURE LIST
   1403 START MEDICAL KNOWLEDGE GENERATION
[Fig. 15]
   1501 PERIOD: JANUARY 1, 2000 TO DECEMBER 31, 2012
   1502 START LINK GENERATION
[Fig. 16]
   1601 PATIENT ID = 1
   HEPATOMA
   LIVER CIRRHOSIS
   SIZE
   1602 LIVER CIRRHOSIS
   SIZE 4 cm OR LESS
   1603 NAME OF DISEASE HEPATOMA
   RATING 1 OR MORE
   DEGREE OF COOCCURRENCE 10 OR MORE
   1604 SEARCH
[Fig. 17]
   1701 KNOWLEDGE NUMBER
   1702 AGGREGATION VALUE
   1703 THRESHOLD
[Fig. 18]
   1801 PRE-AGGREGATION PROCESSING UNIT
   1802 SEARCH PROCESSING UNIT
   1803 SEARCH RESULT OUTPUT UNIT
[Fig. 19]
   1901 COLLECT LINK HAVING WEIGHT OF THRESHOLD OR MORE
   1902 AGGREGATE NUMBER OF LINKS FOR EACH MEDICAL KNOWLEDGE NUMBER
   1903 OUTPUT AGGREGATION VALUE
[Fig. 20]
   2001 ACQUIRE SEARCH CONDITION
   2002 SEARCH NAME OF DISEASE
   2003 PERFORM NARROWING DOWN WITH DEGREE OF COOCCURRENCE AND RATING
   20004 DISPLAY AGGREGATION RESULT OF VIEW AND GRAPH

## Claims

1. An information structuring system configured to structure clinical information using a database in which medical knowledge information including medical concept information is stored,
the medical knowledge information further including a degree of cooccurrence of the medical concept information, and literature rating information of a medical literature including the medical concept information,
the information structuring system comprising:
a clinical information input reception unit configured to receive an input of a plurality of pieces of clinical information; and
a link generation unit configured to generate link information that associates the plurality of pieces of clinical information each other using the medical concept information, the degree of cooccurrence, and the literature rating information.

2. The information structuring system according to claim 1, wherein
the medical knowledge information includes classification information of the medical concept information, and
the link generation unit generates the link information that associates the plurality of pieces of clinical information each other when the clinical information includes the classification information.

3. The information structuring system according to claim 1, further comprising:
a medical concept extraction unit configured to extract the medical concept information from the medical literature information; and
a medical knowledge information generation unit configured to acquire the degree of cooccurrence of the medical concept information and the literature rating information of the medical literature information, and to store the medical concept information, the degree of cooccurrence, and the literature rating information in the database as the medical knowledge information.

4. The information structuring system according to claim 3, wherein
the database stores dictionary information, and
the medical concept extraction unit extracts the medical concept information together with the classification information from the medical literature information using the dictionary information.

5. The information structuring system according to claim 4, wherein
the medical knowledge information generation unit calculates the degree of cooccurrence as a number of the medical concept information included in a literature indicated by the medical literature information.

6. The information structuring system according to claim 4, wherein
the database stores literature rating list information, and
the medical knowledge information generation unit generates the literature rating information of a literature indicated by the medical literature information in which the medical concept information is included using the literature rating list information.

7. The information structuring system according to claim 3, further comprising:
a medical knowledge generation period reception unit configured to receive an input of medical knowledge generation period information,
wherein the medical concept extraction unit selects the medical literature information to be used for the extraction of medical concept based on the medical knowledge generation period information.

8. The information structuring system according to claim 1, further comprising:
a link generation period reception unit configured to receive an input of link generation period information,
wherein the link generation unit generates the link information based on the plurality of pieces of clinical information during period indicated by the link generation period information.

9. The information structuring system according to claim 1, wherein
the link generation unit calculates weight information based on the degree of cooccurrence and the literature rating information, and generates the link information using the weight information,
the information structuring system further comprises: a link information extraction unit configured to extract the link information in which the weight information is a predetermined threshold or more, and
a link information aggregation unit configured to aggregate the degree of concurrence and the literature rating information from the extracted link information and to generate link aggregation information.

10. The information structuring system according to claim 9, further comprising:
a search condition input unit configured to receive an input of a search condition including search cooccurrence information and search literature rating information,
wherein the link information extraction unit searches the link aggregation information based on the search condition, and extracts the link aggregation information that satisfies the search condition.
